# EUROPÄISCHE PATENTSCHRIFT

(11) **EP 2 236 076 B1**
(45) Veröffentlichungstag und Bekanntmachung des Hinweises auf die Patenterteilung: **01.11.2017**
(21) Anmeldenummer: 09004527.9
(22) Anmeldetag: 30.03.2009
(51) Int. Cl.: A61B 5/00, G06F 19/00

(54) **Verfahren und System zur Ermittlung der Differenz zwischen prä- und postprandialen Blutzuckerwerten**
Method and system for calculating the difference between preprandial and postprandial blood sugar values
Procédé et système de détermination de la différence entres des valeurs pré- et postprandiales

(43) Veröffentlichungstag der Anmeldung: 06.10.2010
(73) Patentinhaber: Roche Diabetes Care GmbH, 68305 Mannheim (DE); F. Hoffmann-La Roche AG, 4070 Basel (CH)
(72) Erfinder: Blasberg, Peter, 69469 Weinheim (DE); Koehler, Matthias, 69514 Laudenbach (DE); Kloos, Alfred, 82008 Unterhaching (DE)

(56) Entgegenhaltungen:
- US-A1- 2007 033 074
- US-A1- 2007 179 352
- US-A1- 2008 071 580
- US-A1- 2008 234 943

## Beschreibung

Die Erfindung betrifft ein Verfahren und System zur Ermittlung der Differenz zwischen prä- und post-prandialen Blutzuckerwerten.

Die Frage nach dem Einfluss von Mahlzeiten auf die Höhe des Blutzuckers (nachfolgend auch Blutglukose genannt) bei Diabetikern ist von hoher medizinischer Relevanz. Dies zeigt sich z.B. darin, dass medizinische Fachgesellschaften wie die IDF (International Diabetes Foundation) oder die ADA (American Diabetes Association) Richtlinien zu dieser Thematik veröffentlicht haben (siehe hierzu beispielsweise http://www.idf.org/webdata/docs/Guideline PMG final.pdf oder http://care.diabetes;iournals.org/cgi/reprint/31/Supplement 1/S12).

Die Aufnahme von Mahlzeiten bzw. Kohlenhydraten ist der wichtigste Einflussfaktor für eine Erhöhung des Blutzuckers bei Typ 1 und Typ 2 Diabetes. Erhöhte Blutzuckerspiegel sind der wichtigste Faktor für die Spätkomplikationen des Diabetes, wie Nierenversagen, Erblindung, Myokardinfarkt, Amputationen, etc.

Die Ermittlung des mahlzeitenbezogenen Blutzuckeranstiegs für das Individuum in Abhängigkeit von den Hauptmahlzeiten ist allerdings nur unzureichend gelöst.

Eine Möglichkeit zur Ermittlung der Blutzuckeranstiege stellen experimentelle Tests wie GCT (Glucose Challenge Test) oder OGTT (Oral Glucose Tolerance Test) dar, bei denen der Patient eine definierte Menge an Glucose aufnimmt und der Blutzucker vor und zu definierten Zeiten nach der Aufnahme bestimmt wird. Diese Tests lassen sich nur unter definierten Bedingungen in professionellen Einrichtungen durchrühren, sind zeitaufwändig und für den Patienten belastend. Des weiteren werden diese Test generell ohne den Einfluss anti-diabetischer Therapeutika durchgeführt.

Medizinisch relevant und interessant ist der Einfluss verschiedener Mahlzeiten auf den Blutzucker unter der typischen Medikation des Patienten. Anhand dessen kann festgestellt werden, ob Art der Medikation, Dosis und Zeit der Verabreichung geeignet sind, postprandiale Blutzuckeranstiege in gewissen Grenzen zu halten.

Die Selbstmessung des Blutzuckers ist prinzipiell eine gut geeignete Methode, um die erwähnten Effekte zu quantifizieren. Allerdings sind gewisse Randbedingungen einzuhalten und zu überprüfen, um die Validität der Messwerte sicher zu stellen.

Wichtig ist dabei eine genaue Zuordnung der Blutzuckerwerte zur Mahlzeit vorzunehmen. Insbesondere muss eine Messung unmittelbar vor der Mahlzeit erfolgen, eine weitere Messung muss innerhalb eines bestimmten Zeitfensters nach der Mahlzeit, typischerweise zwischen 1 und max. 2 Stunden nach der Mahlzeit, erfolgen. Des weiteren sollten mehrere Messwertepaare von mehreren Tagen zu verschiedenen Mahlzeiten bzw. Tageszeiten vorliegen, um statistisch solide Aussagen zu den Blutzuckerveränderungen bei verschiedenen Mahlzeiten treffen zu können.

Neben den o. g. klinischen Tests GCT und OGTT steht derzeit nur die Selbstmessung der Blutzuckerwerte zur Verfügung. Die gemessenen Werte können dann z.B. in Diabetes-Tagebücher eingetragen werden. Diese Tagebücher weisen häufig Rubriken wie "vor dem Frühstück" und "nach dem Frühstück" auf, in welche dann die gemessenen Werte eingetragen und durch den Arzt begutachtet werden können. Aus der Literatur ist jedoch bekannt, dass manuell erfasste Blutzuckerwerte zu einem hohen Prozentsatz (> 50%) inkorrekt und unvollständig sind (vgl. z. B. Reliability of self-recorded blood glucose data in patient logbooks compared with SMBG data saved in device memory and printed out with Accu-Chek Smart Printer; J. K. Tshiang Tshiananga, D. Franke, M. Luebker, C. Weber, K. Neeser, 43rd EASD (2007) Annual Meeting, Amsterdam). Darüber hinaus ist eine korrekte Einhaltung der Zeitkriterien für die Messungen nicht nachvollziehbar. Weiter ist eine Auswertung mehrerer Messereignisse nur schwer durchzuführen und ebenso fehleranfällig.

Als Alternative können die Blutzuckerwerte aus dem Speicher des Blutzuckermessgerätes ausgelesen werden und mit entsprechenden Softwareprogrammen analysiert werden. Einige Blutzuckermessgeräte erlauben auch die Markierung einzelner Werte als "prä-" bzw. "postprandial" (d. h. in diesem Zusammenhang als "vor" bzw. "nach" einer Mahlzeit vorgenommen).

Zumeist basieren diese Software-Auswertungen jedoch darauf, dass die gespeicherten Messwerte anhand des Messzeitpunktes in verschiedene Mahlzeitenklassen eingeordnet werden. Wenn die Mahlzeitenklasse "vor dem Frühstück" z.B. von 5:30 Uhr bis 8:00 Uhr definiert ist, wird ein um 5:32 Uhr gemessener Wert automatisch dieser Mahlzeitenklasse zugewiesen. Teilweise werden auch technische Lösungen angeboten, bei denen unabhängig von der Uhrzeit ein Messwert einer bestimmten Mahlzeitenklasse direkt zugewiesen werden kann.

US 2007/0033074 A1 beschreibt ein Diabetes-Datenmanagementsystem. In Figur 6 der US 2007/0033074 A1 wird ein wöchentlicher Logbuch-Bericht mit Blutglukosewerten vor und nach Mahlzeiten offenbart.

US2008/234943 A1 beschreibt mehrere Blutglukosewerte zur Verfügung zu stellen und daraus einen durchschnittlichen Wert betreffend die Anzahl der Messungen pro Tag oder pro Woche zu bestimmen. Liegt die Anzahl der Messungen bei weniger als etwa 3 Messungen pro Woche, wird eine Warnung angezeigt, dass die Anzahl der Messungen nicht ausreicht. Darüber hinaus wird aus der Anzahl von zur Verfügung gestellten Messungen von Blutglukosewerten der Median für post- und prä-Mahlzeitwerte gebildet. Anschließend wird eine Differenz aus den Medianwerten gebildet.

Die Mehrzahl der vorhandenen Software-Programme berechnet durchschnittliche Blutzuckerwerte und Streumasse (z.B. Standard-Abweichung oder Spannweite) für die verschiedenen Mahlzeitenklassen.

Der Nachteil der existierenden Lösungen liegt darin, dass die Art der statistischen Behandlung der gemessenen Blutzuckerwerte eine fehlerhafte Interpretation der mahlzeitenbezogenen Blutzuckerdifferenzen nahelegt.

Durch die Berechnung der isolierten Statistik für die einzelnen Mahlzeitenklassen werden leicht Rückschlüsse über den Mahlzeiteneinfluss anhand von Mittelwertdifferenzen zwischen den ,vor' und 'nach'-Werten (d. h. den prä- und post-prandialen Messwerten) gezogen. Diese können z.B. deshalb irreführend dein, weil die 'vor'-Messungen an anderen Tagen als die 'nach' Messungen erfolgt sind und Unterschiede zwischen den Werten nicht auf die eingenommenen Mahlzeiten zurückzuführen sind. Des weiteren wird in aller Regel außer acht gelassen, dass für eine korrekte Ermittlung der Mahlzeiten-bezogenen Blutzuckerveränderung eine korrekte zeitliche Abfolge der Blutzuckermessungen in Relation zur Mahlzeitenaufhahme gegeben sein muss.

Aufgabe der Erfindung ist es, die Nachteile des Standes der Technik zu beseitigen. Insbesondere soll die vorliegende Erfindung ein Verfahren und System bereitstellen, dass eine weniger fehleranfällige, vorzugsweise automatisierte Zuordnung von zu einer Mahlzeit gehörenden prä- und postprandialen Blutglukosewerten ermöglicht. So sollen verbesserte Auswertemöglichkeiten und damit verbundene medizinische Rückschlüsse ermöglicht werden.

Die Aufgabe wird durch den Gegenstand der Erfindung gelöst.

Gegenstand der Erfindung ist ein Verfahren gemäß Anspruch 1, ein System gemäß Anspruch 8 und ein Computerprogrammprodukt gemäß Anspruch 9. Bevorzugte Ausführungsformen der Erfindung sind Gegenstand der abhängigen Ansprüche.

Voraussetzung für das nachfolgend beschriebene Verfahren zur Ermittlung mahlzeitenbezogener Blutzuckerwertveränderungen ist die Kennzeichnung von Blutzuckerwerten als ,vor' oder ,nach' der Mahlzeit. Diese Kennzeichnung erfolgt am Blutzuckermessgerät unmittelbar im Zusammenhang mit der Blutzuckermessung. Eine Vielzahl von derzeit kommerziell erhältlichen Blutglukosemessgeräten erlaubt diese Markierung durch entsprechende Eingabe über Bedienknöpfe am Messgerät.

Die in einem Blutglukosemessgerät gespeicherten Blutglukosemesswerte können nun mit einer relativen (d. h. z. B. von einem internen Zeitzähler ermittelten, der beispielsweise die relative Zeit seit der Herstellung oder Erstinbetriebnahme des Messgeräts ermittelt) oder absoluten (d. h. z. B. mit Datum und Uhrzeit)Zeitmarke versehen werden und zusammen mit diesen Markierungen, die auch als "prä- bzw. postprandiales Flagging" bezeichnet werden, an ein geeignetes Auswerteprogramm übertragen werden. Das Programm kann dabei im Blutzuckermessgerät selbst enthalten sein oder in einem anderen, zur Datenverarbeitung geeigneten Gerät, wie z. B. Computer, Mobiltelefon, PDA oder ähnlichem, laufen.

In einer alternativen Ausführungsform der Erfindung ist es möglich, einen unmarkierten bzw. irrtümlich als prä-prandial markierten Wert innerhalb einer bestimmten, vordefinierten Zeitfensters, z. B. 1,5h nach einem als prä-prandial markierten Wert automatisch bzw. nach Rückfrage als post-prandial markierten Wert zu interpretieren. Typischerweise wird dieser Wert dann nachträglich als "nach"-Blutzuckermesswert markiert.

Erfindungsgemäß werden Mahlzeit-induzierte Blutzuckerveränderungen wie folgt ermittelt:
Die Daten werden, beginnend mit dem ältesten Wert, nach Werten durchsucht, die die Markierung ,vor' tragen, d. h. als prä-prandiale Werte gekennzeichnet sind.

Ausgehend von der Uhrzeit eines gefundenen 'vor'-Wertes wird in einem bestimmten Zeitfenster, typischerweise von minimal 60 Minuten und maximal 120 Minuten nach dem "vor"-Wert, nach Blutzuckerwerten mit der Markierung ,nach' gesucht. Werden in diesem Zeitabschnitt mehrere Blutzuckerwerte mit der 'nach'-Markierung gefunden, wird für nachfolgende Berechnung einer der "nach"-Werte, vorzugsweise der höchste der gefundenen Messwerte, verwendet.

Es wird die Differenz (,nach' minus ,vor') des gefundenen Paares ermittelt und typischerweise unter einer von vier Zeitrubriken ('Frühstück', 'Mittagessen', 'Abendessen' sowie 'Abend /Nacht') gespeichert.

Die entsprechende Zeitrubrik wird anhand der Messzeit des 'vor'-Wertes ermittelt. Die Zeitblöcke (vor / nach Frühstück, vor / nach Mittagessen, vor /nach Abendessen sowie Abend / Nacht) lassen sich bevorzugt individuell einstellen.

Wurde ein vor / nach Paar ermittelt und gespeichert, wird typischerweise die Suche nach weiteren Paaren nach dem zuvor gefundenen Paar (d. h. nach der Messzeit des zuvor gefundenen 'nach'-Wertes) fortgesetzt, bis alle Werte bis zum jüngsten Blutzuckermesswert durchsucht sind.

Anschließend werden die Anzahl, der Mittelwert und die Standardabeichung für alle gefundenen Paare sowie für die genannten Zeitblöcke ermittelt und ausgegeben.

Durch das erfindungsgemäße Verfahren wird sichergestellt, dass jeweils zu einer bestimmten Mahlzeit gehörende Blutzuckermesswertepaare miteinander verglichen werden. Dadurch wird eine zuverlässigere Aussage über den Einfluss einzelner Mahlzeiten auf den Blutzuckerwert und den Effekt der Therapiemaßnahmen (z. B. Insulingabe durch Spritzen, Pens oder Insulinpumpen, orale Einnahme von Blutzucker senkenden Medikamenten) ermöglicht.

Die Erfindung soll anhand der Figur näher erläutert werden.

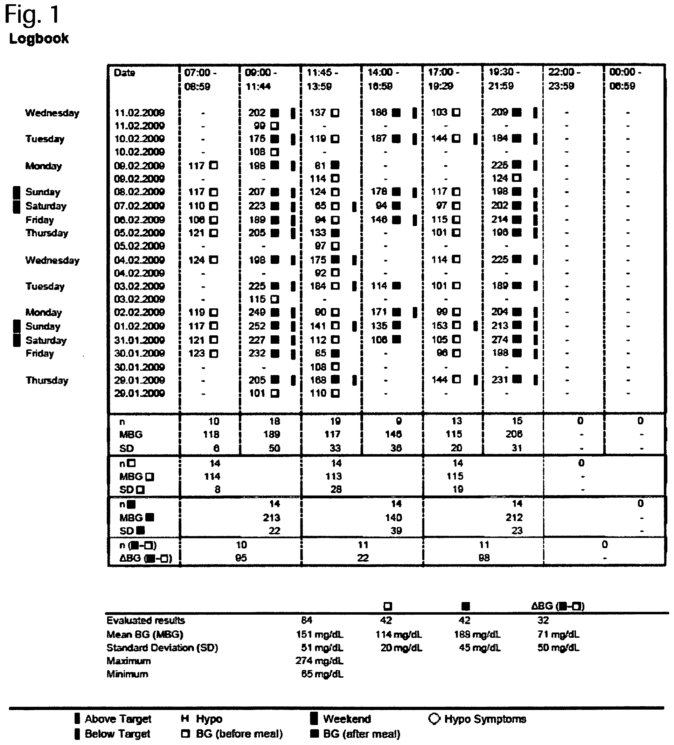

Figur 1 zeigt exemplarisch anhand eines Reports eine mögliche Darstellungsform von Blutglukosemessdaten.

Die vor bzw. nach Mahlzeiten ermittelten Blutglukosemesswerte sind durch den Benutzer des Messsystems markiert worden und werden in der Darstellung der Figur 1 mit leeren Quadraten (vor einer Mahlzeit ermittelter Wert) und vollen Quadraten (nach einer Mahlzeit ermittelter Wert) gekennzeichnet. Im oberen, tabellenartigen Bereich der Figur 1 sind die eigentlichen Messdaten mit Wochentag, Datum, Uhrzeitbereich und "vor"- bzw. "nach"-Markierung dargestellt. In der Tabelle sind neben allgemeinen statistischen Werten auch die Werte für die Anzahl n der "vor" und "nach"-Blutzuckermesswerte (d. h. der prä- und prostprandialen Blutglukosemesswerte), ihre Durchschnittswerte (MBG für engl. "mean blood glucose value"), Standardabweichungen (SD für engl. "standard deviation") sowie die Differenzwerte für zueinander gehörige prä- und prostprandialen Blutglukosemesswertepaare (ΔBG) in den jeweiligen Zeitblöcken dargestellt. Eine Zusammenfassung der Daten für alle Messwerte findet sich im darunterliegenden Abschnitt. Hier ist auch ersichtlich, dass bei der Anwendung des erfindungsgemäßen Verfahrens nicht für alle präprandialen Messwerte dazugehörige postprandiale Werte gefunden wurden, da die Anzahl der Wertepaare in der Rubrik ΔRG nur 32 beträgt, insgesamt aber 42 prä- und 42 postprandialen Werte gemessen wurden.

Das erfindungsgemäße Verfahren und System ermöglicht ein einfaches Erkennen und Darstellen der sogenannten Meal-Excursions, d. h. des Anstiegs oder Abfalls des Blutzuckerwertes nach der Einnahme von Mahlzeiten. Dies wiederum erlaubt eine vereinfachte und verbesserte Kontrolle der Insulintherapie und ggf. eine Anpassung von Dosis und Zeitpunkt von mahlzeitenbezogenen Insulingaben und so schlussendlich eine Verbesserung der Therapie und eine Verringerung oder Vermeidung von Diabetesspätfolgen.

## Patentansprüche

1. Verfahren zur Ermittlung des Unterschieds zwischen präprandialen und postprandialen Blutglukosemesswerten umfassend die Schritte
i) Auslesen von mit einer relativen oder absoluten Zeitmarke versehenen und als präprandial gekennzeichneten Blutglukosemesswerten und von mit einer relativen oder absoluten Zeitmarke versehenen postprandialen Blutglukosemesswerten aus dem Speicher eines Blutglukosemessgeräts,
ii) Ermitteln der zeitlichen Reihenfolge der als präprandial markierten Blutglukosemesswerte,
iii) Ermitteln der zu den jeweiligen präprandialen Blutglukosemesswerte korrespondierenden postprandialen Blutglukosemesswerte durch Anwenden eines zeitlichen Auswahlkriteriums,
iv) Ermitteln der Differenz zwischen korrespondierenden prä- und postprandialen Blutglukosemesswerten, und
v) Anzeigen der Differenzwerte.

2. Verfahren gemäß Anspruch 1, **dadurch gekennzeichnet, dass**
die Blutglukosemesswerte und die Differenz zwischen korrespondierenden prä- und postprandialen Blutglukosemesswerten Zeitrubriken zugeordnet werden, die Mahlzeiten repräsentieren.

3. Verfahren gemäß Anspruch 2, **dadurch gekennzeichnet, dass**
die Zuordnung zu den Zeitrubriken anhand der Uhrzeit des präprandialen Blutglukosemesswertes erfolgt.

4. Verfahren gemäß einem der Ansprüche 1 bis 3, **dadurch gekennzeichnet, dass** in Schritt ii) die Sortierung aufsteigend beginnend mit dem ältesten präprandialen Blutglukosemesswert erfolgt.

5. Verfahren gemäß einem der Ansprüche 1 bis 4, **dadurch gekennzeichnet, dass** in Schritt iii) als zeitliches Auswahlkriterium ein Zeitfenster von mindestens 60 Minuten und höchstens 120 Minuten nach dem jeweiligen präprandialen Blutglukosemesswert angewandt wird.

6. Verfahren gemäß einem der Ansprüche 1 bis 5, **dadurch gekennzeichnet, dass** im Falle des Vorliegens mehrerer postprandialer Blutglukosemesswerte, die das zeitliche Auswahlkriterium erfüllen, der jeweils höchste Blutglukosemesswert in Schritt iv) verwendet wird.

7. Verfahren gemäß einem der Ansprüche 1 bis 6, **dadurch gekennzeichnet, dass** die Schritte ii) und iii) dergestalt ablaufen, dass
ausgehend vom ältesten präprandialen Blutglukosemesswert zunächst der höchste korrespondierende postprandiale Blutglukosemesswert ermittelt wird,
dieses Blutglukosemesswertepaar zur Weiterverarbeitung gespeichert wird,
anschließend der nächstjüngere präprandiale Blutglukosemesswert und der höchste korrespondierende postprandiale Blutglukosemesswert ermittelt wird,
dieses Blutglukosemesswertepaar zur Weiterverarbeitung ebenfalls gespeichert wird, und diese Schritte wiederholt werden, bis alle Blutglukosemesswerte bis zum jüngsten präprandialen Blutglukosemesswert und gegebenenfalls dem höchsten korrespondierende postprandialen Blutglukosemesswert berücksichtigt sind.

8. System zur Ermittlung des Unterschieds zwischen präprandialen und postprandialen Blutglukosemesswerten umfassend eine Datenverarbeitungseinrichtung und ein Computerprogramm zur Ausführung des Verfahrens gemäß einem der Ansprüche 1 bis 7.

9. Computerprogrammprodukt zur Ermittlung des Unterschieds zwischen präprandialen und postprandialen Blutglukosemesswerten enthaltend ein Computerprogramm zur Ausführung des Verfahrens gemäß einem der Ansprüche 1 bis 7.

## Claims

1. Method for determining the difference between preprandial and postprandial blood glucose measurement values comprising the steps:
i) reading blood glucose measurement values identified as preprandial that are provided with a relative or absolute time mark and postprandial blood glucose measurement values that are provided with a relative or absolute time mark from the memory of a blood glucose measuring device,
ii) determining the chronological sequence of the blood glucose measurement values marked as preprandial,
iii) determining by using a chronological selection criterion the postprandial blood glucose measurement values that correspond to the respective preprandial blood glucose measurement values,
iv) determining the difference between corresponding preprandial and postprandial blood glucose measurement values, and
v) displaying the difference values.

2. Method according to claim 1, **characterised in that**
the blood glucose measurement values and the difference between the corresponding preprandial and postprandial blood glucose measurement values are assigned to time categories that represent meal times.

3. Method according to claim 2, **characterised in that**
the assignment to the categories is carried out based on the time of day of the preprandial blood glucose measurement value.

4. Method according to any of claims 1 to 3, **characterised in that**
in step ii) the sorting takes place in ascending order starting with the oldest preprandial blood glucose measurement value.

5. Method according to any of claims 1 to 4, **characterised in that**
in step iii) a time window of at least 60 minutes and at most 120 minutes after the respective preprandial blood glucose measurement value is used as the chronological selection criterion.

6. Method according to any of claims 1 to 5, **characterised in that** in the case that a plurality of postprandial blood glucose measurement values that fulfil the chronological selection criterion are present, the respectively highest blood glucose measurement value in step iv) is used.

7. Method according to any of claims 1 to 6, **characterised in that**
the steps ii) and iii) take place in such a manner that
firstly the highest corresponding postprandial blood glucose measurement value is determined starting from the oldest preprandial blood glucose measurement value,
this pair of blood glucose measurement values is stored for further processing,
then the next earlier preprandial blood glucose measurement value and the highest corresponding postprandial blood glucose measurement value are determined,
this pair of blood glucose measurement values is likewise stored for further processing, and these steps are repeated until all the blood glucose measurement values up to the most recent preprandial blood glucose measurement value and, where appropriate, the highest corresponding postprandial blood glucose measurement value are considered.

8. System for determining the difference between preprandial and postprandial blood glucose measurement values comprising a data processing device and a computer program for carrying out the method according to any of claims 1 to 7.

9. Computer program product for determining the difference between preprandial and postprandial blood glucose measurement values containing a computer program for carrying out the method according to any of claims 1 to 7.

## Revendications

1. Procédé de détermination de la différence entre des valeurs de glycémie préprandiales et postprandiales comprenant les étapes consistant à
i) lire les valeurs de glycémie munies d'un horodatage relatif ou absolu et marquées comme étant préprandiales et lire les valeurs de glycémie postprandiales munies d'un horodatage relatif ou absolu à partir de la mémoire d'un appareil de mesure de la glycémie,
ii) déterminer la séquence chronologique des valeurs de glycémie marquées comme étant préprandiales,
iii) déterminer les valeurs de glycémie postprandiales qui correspondent à chacune des valeurs de glycémie préprandiales en appliquant un critère de sélection temporel,
iv) déterminer la différence entre les valeurs de glycémie préprandiales et postprandiales correspondantes et
v) afficher la valeur des différences.

2. Procédé selon la revendication 1, **caractérisé en ce que**
les valeurs de glycémie et la différence entre les valeurs de glycémie préprandiales et postprandiales correspondantes sont classées en catégories temporelles représentant les repas.

3. Procédé selon la revendication 2, **caractérisé en ce que**
le classement dans les catégories temporelles s'effectue en fonction de l'heure de la valeur de glycémie préprandiale.

4. Procédé selon l'une des revendications 1 à 3, **caractérisé en ce que**,
à l'étape ii), le tri s'effectue dans l'ordre croissant en commençant par la valeur de glycémie préprandiale la plus ancienne.

5. Procédé selon l'une des revendications 1 à 4, **caractérisé en ce qu'**on applique,
à l'étape iii), un laps de temps d'au moins 60 minutes et d'au plus 120 minutes après la valeur de glycémie préprandiale respective, en tant que critère de sélection temporel.

6. Procédé selon l'une des revendications 1 à 5, **caractérisé en ce qu'**on utilise,
dans le cas où plusieurs valeurs de glycémie postprandiales répondant au critère de sélection temporel sont présentes, la valeur de glycémie respectivement la plus haute à l'étape iv).

7. Procédé selon l'une des revendications 1 à 6, **caractérisé en ce que**
les étapes ii) et iii) se déroulent de manière à
déterminer tout d'abord la valeur de glycémie postprandiale la plus haute correspondante à partir de la valeur de glycémie préprandiale la plus ancienne ; mémoriser ces deux valeurs de glycémie pour une utilisation ultérieure ;
déterminer ensuite la valeur de glycémie préprandiale suivante et la valeur de glycémie postprandiale la plus haute correspondante ;
mémoriser également ces deux valeurs de glycémie pour une utilisation ultérieure et répéter ces étapes jusqu'à prendre en compte toutes les valeurs de glycémie jusqu'à la valeur de glycémie préprandiale la plus récente et, le cas échéant, la valeur de glycémie postprandiale la plus haute correspondante.

8. Système pour déterminer la différence entre des valeurs de glycémie préprandiales et postprandiales, comprenant un dispositif de traitement des données et un programme informatique pour mettre en oeuvre le procédé selon l'une des revendications 1 à 7.

9. Progiciel informatique pour déterminer la différence entre des valeurs de glycémie préprandiales et postprandiales, comprenant un programme informatique pour mettre en oeuvre le procédé selon l'une des revendications 1 à 7.
